# EUROPEAN PATENT SPECIFICATION

(11) **EP 1 599 445 B1**
(45) Date of publication and mention of the grant of the patent: **02.04.2008**
(21) Application number: 04713874.8
(22) Date of filing: 24.02.2004
(51) Int. Cl.: C07D 207/26, C07D 207/38, A61K 31/40

(54) **COMPOUNDS HAVING ACTIVITY AT 5HT2C RECEPTOR AND USES THEREOF**
AN 5HT2C-REZEPTOR AKTIVE VERBINDUNGEN UND IHRE VERWENDUNGEN
COMPOSES AYANT UNE ACTIVITE SUR LE RECEPTEUR 5HT2C ET LEURS UTILISATIONS

(30) Priority: 05.03.2003 GB 0305024
(43) Date of publication of application: 30.11.2005
(73) Proprietor: GLAXO GROUP LIMITED, Greenford, Middlesex UB6 ONN (GB)
(72) Inventor: DAMIANI, Federica, 37135 Verona (IT); HAMPRECHT, Dieter, 37135 Verona (IT); MICHELI, Fabrizio, 37135 Verona (IT); PASQUARELLO, Alessandra, 37135 Verona (IT); TEDESCO, Giovanna, 37135 Verona (IT)
(74) Representative: Thompson, Clive Beresford
(86) International application number: PCT/EP2004/001843
(87) International publication number: WO 2004/078718

(56) References cited:
- WO-A-01/96308
- WO-A-97/48700
- WO-A-03/057220
- WO-A-03/089409

## Description

This invention relates to novel compounds having pharmacological activity, processes for their preparation, to compositions containing them and to their use in the treatment of CNS and other disorders.

WO 96/23783, WO 97/48699 and WO 97/48700 all disclose a series of indoline derivatives which are 5-HT_{2C} receptor antagonists and which are claimed to be useful in the treatment of various CNS disorders.

WO 01/96308 discloses pyridinone compounds useful for the treatment of neurodegenerative and CNS related disorders.

None of the above references disclosed compounds falling into the scope of the present invention.

A novel class of compounds possessing 5-HT_{2C} receptor activity has been found. The present invention therefore provides, in a first aspect, a compound of formula (I) or a pharmaceutically acceptable salt thereof:
wherein: 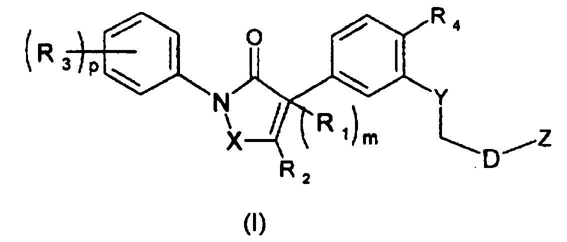
- R₁: is hydrogen;
- m: is 0 when = is a double bond and m is 1 when = is a single bond;
- R₂: is hydrogen, C₁₋₆alkyl;
- X: is a group -(CHR₅)- wherein R₅ is hydrogen, C₁₋₆alkoxy;
- R₃: is halogen, C₁₋₆alkoxy;
- p is: 1 or 2;
- R₄: is C₁₋₆alkoxy, OCF₃, halogen or cyano,;
- Y is: oxygen;
- D is: -CH₂-; and
- Z is: is pyperidyl.

The following terms, whether used alone or as part of another group are to be given the following meanings, unless otherwise stated.

The term "halogen" and its abbreviated form "halo" are used herein to describe fluorine, chlorine, bromine or iodine.

The term "alkyl" is used herein to describe a straight chain or branched fully saturated hydrocarbon group. "C₁₋₆alkyl" refers to alkyl groups having from one to six carbon atoms, including all isomeric forms, such as methyl, ethyl, propyl, isopropyl, butyl, isobutyl, sec-butyl, tert-butyl, pentyl, neopentyl, sec-pentyl, n-pentyl, isopentyl, tert-pentyl and hexyl.

The term "C₁₋₆alkoxy" refers to a straight chain or branched chain alkoxy (or "alkyloxy") group having from one to six carbon atoms, including all isomeric forms, such as methoxy, ethoxy, propoxy, isopropoxy, butoxy, isobutoxy, sec-butoxy, tert-butoxy, pentoxy, neopentoxy, sec-pentoxy, n-pentoxy, isopentoxy, tert-pentoxy and hexoxy.

Preferably X is -CH₂-.

Preferably R₂ is hydrogen.

Preferably R₃ is/are particularly chloro or fluoro, attached at the 3 or the 3,4-positions of the phenyl ring.

Preferred compounds are compounds when = is a single bond.

Preferred compounds are compounds of formula (Ia): 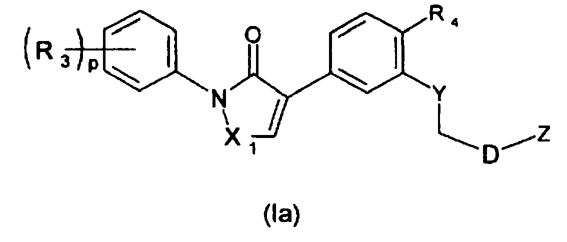 wherein R₃, p, R₄, Y, D, Z, = are as defined for formula (I) and X₁ is -CH₂- or - HC(OH)-. Preferred features of formula (I) also apply to formula (Ia).

Preferred compounds include:
1. 1-(3,4-Dichloro-phenyl)-5-hydroxy-3-[4-methoxy-3-(2-piperidin-1-yl-ethoxy)-phenyl]-pyrrolidin-2-one
2. 1-(3,4-Dichloro-phenyl)-3-[4-methoxy-3-(2-piperidin-1-yl-ethoxy)-phenyl]-1,5-dihydro-pyrrole-2-one
3. 1-(3,4-Dichloro-phenyl)-3-[4-methoxy-3-(2-piperidin-1-yl-ethoxy)-phenyl]-pyrrolidin-2-one
4. 1-(3,4-Dichloro-phenyl)-5-methoxy-3-[4-methoxy-3-(2-piperidin-1-yl-ethoxy)-phenyl]-pyrrolidin-2-one
5. 1-(4-Methoxyphenyl)-3-[4-methoxy-3-(2-piperidin-1-yl-ethoxy)-phenyl]-1,5-dihydropyrrole-2-one
6. 1-(4-Methoxyphenyl)-3-[4-methoxy-3-(2-piperidin-1-yl-ethoxy)-phenyl]-pyrrolidin-2-one
7. 1-(3-Chloro-4-methoxy-phenyl)-3-[4-methoxy-3-(2-piperidin-1-yl-ethoxy)-phenyl]-pyrrolidin-2-one
8. 1-(3-Chloro-4-methoxy-phenyl)-3-[4-methoxy-3-(2-piperidin-1-yl-ethoxy)-phenyl]-1,5-dihydro-pyrrole-2-one
9. 1-(3-Fluoro-4-methoxy-phenyl)-3-[4-methoxy-3-(2-piperidin-1-yl-ethoxy)-phenyl]-pyrrolidin-2-one
10. 1-(3-Fluoro-4-methoxy-phenyl)-3-[4-methoxy-3-(2-piperidin-1-yl-ethoxy)-phenyl]-1,5-dihydro-pyrrole-2-one
11. 1-(3-Fluoro-phenyl)-3-[4-methoxy-3-(2-piperidin-1-yl-ethoxy)-phenyl]-pyrrolidin-2-one
12. 1-(3-Fluoro-phenyl)-3-[4-methoxy-3-(2-piperidin-1-yl-ethoxy)-phenyl]-1,5-dihydropyrrole-2-one
13. 1-(3,4-Dichloro-phenyl)-5,5-dimethyl-4-hydroxy-3-[4-methoxy-3-(2-piperidin-1-yl-ethoxy)-phenyl]-pyrrol-2-one
14. 1-(3,4-Dichloro-phenyl)-5,5-dimethyl-4-hydroxy-3-[4-methoxy-3-(2-piperidin-1-yl-ethoxy)-phenyl]-pyrrol-2-one
and pharmaceutically acceptable salts thereof.

The compounds of formula (I) can form acid addition salts. It will be appreciated that for use in medicine the salts of the compounds of formula (I) should be pharmaceutically acceptable. Suitable pharmaceutically acceptable salts will be apparent to those skilled in the art and include those described in J. Pharm. Sci., 1977, 66, 1-19, such as acid addition salts formed with inorganic acids e.g. hydrochloric, hydrobromic, sulfuric, nitric or phosphoric acid; and organic acids e.g. succinic, maleic, acetic, fumaric, citric, tartaric, benzoic, p-toluenesulfonic, methanesulfonic or naphthalenesulfonic acid.

The compounds of this invention may be in crystalline or non-crystalline form, and, if crystalline, may optionally be hydrated or solvated. This invention includes within its scope stoichiometric hydrates as well as compounds containing variable amounts of water.

Certain compounds of formula (I) are capable of existing in stereoisomeric forms (e.g. geometric or *("cis-trans")* isomers, diastereomers and enantiomers) and the invention extends to each of these stereoisomeric forms and to mixtures thereof including racemates. The different stereoisomeric forms may be separated one from the other by the usual methods, or any given isomer may be obtained by stereospecific or asymmetric synthesis. The invention also extends to any tautomeric forms and mixtures thereof.

The present invention also provides a process for the preparation of a compound of formula (I) or a pharmaceutically acceptable salt thereof, which process comprises:
(a) reacting a compound of formula (II): 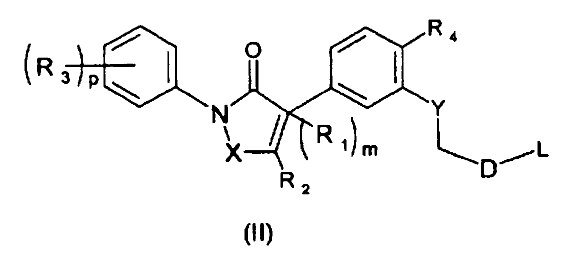 wherein R₁, R₂, R₃, R₄, m. p, X, =, Y and D are as defined for formula (I), and L is a leaving group, with a compound of formula (III):

   Z-H (III)

   wherein Z is as defined for formula (I); or
(b) cyclising a compound of formula (IV): 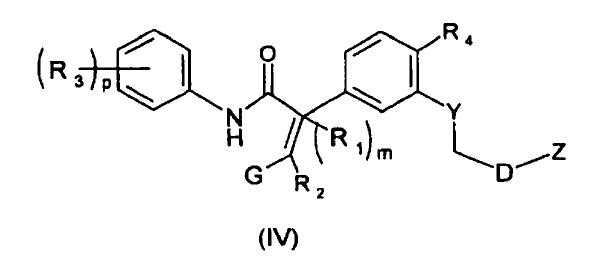
wherein R₁, R₂, m, R₃, p, R4, Y, D, Z and = are as defined for formula (I) and G is a group -X=CH₂, wherein X is as defined for formula (I), dehydrogenated as required;
and thereafter, for either process (a) or process (b), optionally followed by:
- removing any protecting groups; and/or
- converting a compound of formula (I) into another compound of formula (I); and/or
- forming a pharmaceutically acceptable salt.

For the reaction of process (a), suitably L is mesylate. The reaction may take place in a solvent such as DMF in the presence of sodium iodide and potassium carbonate.

The reaction of process (b) suitably takes place in a solvent such as THF in the presence of OsO₄ and NalO₄.

Compounds of formula (I) can be converted into further compounds of formula (I) using standard techniques. For example, and by way of illustration rather than limitation, a compound wherein X is -(HCOH)- may be converted to a compound wherein X is -(CH₂)-by using a suitable reducing agent such as triethylsilane-trifluoroacetic acid using dichloromethane as solvent, and a compound wherein R₁ is hydroxy may be converted to compound wherein m is 0 and = is a double bond by an elimination reaction in TFA.

Compounds of formulae (II), (III) and (IV) are commercially available or may be prepared according to methods described herein or may be prepared according to known methods or by analogous methods thereto.

Those skilled in the art will appreciate that it may be necessary to protect certain groups to carry out the above processes. Suitable protecting groups and methods for their attachment and removal are conventional in the art of organic chemistry, such as those described in Greene T.W. 'Protective groups in organic synthesis' New York, Wiley (1981).

Pharmaceutically acceptable salts may be prepared conventionally by reaction with the appropriate acid or acid derivative.

In another aspect, the present invention provides a pharmaceutical composition comprising a compound of formula (I) or a pharmaceutically acceptable salt thereof, and a pharmaceutically acceptable carrier or excipient.

In a further aspect, the present invention provides a process for preparing a pharmaceutical composition, the process comprising mixing a compound of formula (I) or a pharmaceutically acceptable salt thereof and a pharmaceutically acceptable carrier or excipient.

A pharmaceutical composition of the invention, which may be prepared by admixture, suitably at ambient temperature and atmospheric pressure, is usually adapted for oral, parenteral or rectal administration and, as such, may be in the form of tablets, capsules, oral liquid preparations, powders, granules, lozenges, reconstitutable powders, injectable or infusible solutions or suspensions or suppositories. Orally administrable compositions are generally preferred.

Tablets and capsules for oral administration may be in unit dose form, and may contain conventional excipients, such as binding agents (e.g. pregelatinised maize starch, polyvinylpyrrolidone or hydroxypropyl methylcellulose);, fillers (e.g. lactose, microcrystalline cellulose or calcium hydrogen phosphate);, tabletting lubricants lubricants (e.g. magnesium stearate, talc or silica);, disintegrants (e.g. potato starch or sodium starch glycollate); and acceptable wetting agents (e.g. sodium lauryl sulphate). The tablets may be coated according to methods well known in normal pharmaceutical practice.

Oral liquid preparations may be in the form of, for example, aqueous or oily suspension, solutions, emulsions, syrups or elixirs, or may be in the form of a dry product for reconstitution with water or other suitable vehicle before use. Such liquid preparations may contain conventional additives such as suspending agents (e.g. sorbitol syrup, cellulose derivatives or hydrogenated edible fats), emulsifying agents (e.g. lecithin or acacia), non-aqueous vehicles (which may include edible oils e.g. almond oil, oily esters, ethyl alcohol or fractionated vegetable oils), preservatives (e.g. methyl or propyl-p-hydroxybenzoates or sorbic acid), and, if desired, conventional flavourings or colorants, buffer salts and sweetening agents as appropriate. Preparations for oral administration may be suitably formulated to give controlled release of the active compound.

For parenteral administration, fluid unit dosage forms are prepared utilising a compound of the invention or pharmaceutically acceptable salt thereof and a sterile vehicle. Formulations for injection may be presented in unit dosage form e.g. in ampoules or in multi-dose, utilising a compound of the invention or pharmaceutically acceptable salt thereof and a sterile vehicle, optionally with an added preservative. The compositions may take such forms as suspensions, solutions or emulsions in oily or aqueous vehicles, and may contain formulatory agents such as suspending, stabilising and/or dispersing agents. Alternatively, the active ingredient may be in powder form for constitution with a suitable vehicle, e.g. sterile pyrogen-free water, before use. The compound, depending on the vehicle and concentration used, can be either suspended or dissolved in the vehicle. In preparing solutions, the compound can be dissolved for injection and filter sterilised before filling into a suitable vial or ampoule and sealing. Advantageously, adjuvants such as a local anaesthetic, preservatives and buffering agents are dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilisation cannot be accomplished by filtration. The compound can be sterilised by exposure to ethylene oxide before suspension in a sterile vehicle. Advantageously, a surfactant or wetting agent is included in the composition to facilitate uniform distribution of the compound.

Lotions may be formulated with an aqueous or oily base and will in general also contain one or more emulsifying agents, stabilising agents, dispersing agents, suspending agents, thickening agents, or colouring agents. Drops may be formulated with an aqueous or non-aqueous base also comprising one or more dispersing agents, stabilising agents, solubilising agents or suspending agents. They may also contain a preservative.

The compounds of the invention may also be formulated in rectal compositions such as suppositories or retention enemas, e.g. containing conventional suppository bases such as cocoa butter or other glycerides.

The compounds of the invention may also be formulated as depot preparations. Such long acting formulations may be administered by implantation (for example subcutaneously or intramuscularly) or by intramuscular injection. Thus, for example, the compounds of the invention may be formulated with suitable polymeric or hydrophobic materials (for example as an emulsion in an acceptable oil) or ion exchange resins, or as sparingly soluble derivatives, for example, as a sparingly soluble salt.

For intranasal administration, the compounds of the invention may be formulated as solutions for administration via a suitable metered or unitary dose device or alternatively as a powder mix with a suitable carrier for administration using a suitable delivery device. Thus compounds of formula (I) may be formulated for oral, buccal, parenteral, topical (including ophthalmic and nasal), depot or rectal administration or in a form suitable for administration by inhalation or insufflation (either through the mouth or nose).

The compounds of the invention may be formulated for topical administration in the form of ointments, creams, gels, lotions, pessaries, aerosols or drops (e.g. eye, ear or nose drops). Ointments and creams may, for example, be formulated with an aqueous or oily base with the addition of suitable thickening and/or gelling agents. Ointments for administration to the eye may be manufactured in a sterile manner using sterilised components.

The compounds of the present invention have affinity for the 5-HT_{2C} receptor. The affinity can be determined by assessing their ability to displace [³H]-mesulergine from rat or human 5-HT_{2C} clones expressed in 293 cells *in vitro,* as described in WO 94/04533.

All the Example compounds were tested according to this assay and were found to have pKi values >5.8. Some compounds show a considerably higher affinity in the range of 7.0 to >9.0 in human cells.

The intrinsic activity of the compounds of this invention can be determined according to the [³⁵S]GTPγS functional assay which is described in WO 99/07700.

Compounds of formula (I) and their pharmaceutically acceptable salts are of use in the treatment of certain CNS disorders such as depression (which term is used herein to include bipolar depression, unipolar depression, single or recurrent major depressive episodes with or without psychotic features, catatonic features, melancholic features, atypical features or postpartum onset, seasonal affective disorder, dysthymic disorders with early or late onset and with or without atypical features, neurotic depression and social phobia, depression accompanying dementia for example of the Alzheimer's type, vascular dementia with depressed mood, schizoaffective disorder or the depressed type, and depressive disorders resulting from general medical conditions including, but not limited to, myocardial infarction, diabetes, miscarriage or abortion, *etc*), anxiety including generalised anxiety and social anxiety disorder, schizophrenia, panic disorder, agoraphobia, social phobia, epilepsy, obsessive compulsive disorder and post-traumatic stress disorder, pain (particularly neuropathic pain), migraine, memory disorders, including dementia, amnesic disorders and age-associated memory impairment, disorders of eating behaviours including anorexia nervosa and bulimia nervosa, sexual dysfunction, sleep disorders (including disturbances of circadian rhythm, dyssomnia, insomnia, sleep apnea and narcolepsy), withdrawal from abuse of drugs such as of cocaine, ethanol, nicotine, benzodiazepines, alcohol, caffeine, phencyclidine (phencyclidine-like compounds), opiates (e.g. cannabis, heroin, morphine), sedative ipnotic, amphetamine or amphetamine-related drugs (e.g. dextroamphetamine, methylamphetamine) or a combination thereof, Alzheimer's disease, motor disorders such as Parkinson's disease, dementia in Parkinson's disease, neuroleptic-induced Parkinsonism and tardive dyskinesias, as well as other psychiatric disorders, disorders associated with spinal trauma and/or head injury such as hydrocephalus, gastrointestinal disorders such as IBS (Irritable Bowel Syndrome), Crohn's disease, ulcerative colitis, non-steroidal antiinflammatory drug induced damage) as well as microvascular diseases such as macular oedema and retinopathy.

It is to be understood that, as used herein, the term "treatment" refers to alleviation of established symptoms as well as prophylaxis.

Thus the present invention also provides a compound of formula (I) or a pharmaceutically acceptable salt thereof, for use as a therapeutic substance. In particular, the present invention provides a compound of formula (I) or a pharmaceutically acceptable salt thereof for use in the treatment of the above disorders. In particular the invention provides a compound of formula (I) or a pharmaceutically acceptable salt thereof for use as a therapeutic substance in the treatment of a CNS disorder. Preferably the CNS disorder is depression or anxiety.

Compounds of the invention may be administered in combination with other active substances such as 5HT3 antagonists, NK-1 antagonists, serotonin agonists, selective serotonin reuptake inhibitors (SSRI), noradrenaline re-uptake inhibitors (SNRI), tricyclic antidepressants and/or dopaminergic antidepressants.

Suitable 5HT3 antagonists which may be used in combination of the compounds of the inventions include for example ondansetron, granisetron, metoclopramide.

Suitable serotonin agonists which may be used in combination with the compounds of the invention include sumatriptan, rauwolscine, yohimbine, metoclopramide.

Suitable SSRIs which may be used in combination with the compounds of the invention include fluoxetine, citalopram, femoxetine, fluvoxamine, paroxetine, indalpine, sertraline, zimeldine.

Suitable SNRIs which may be used in combination with the compounds of the invention include venlafaxine and reboxetine.

Suitable tricyclic antidepressants which may be used in combination with a compound of the invention include imipramine, amitriptiline, chlomipramine and nortriptiline.

Suitable dopaminergic antidepressants which may be used in combination with a compound of the invention include bupropion and amineptine.

It will be appreciated that the compounds of the combination or composition may be administered simultaneously (either in the same or different pharmaceutical formulations), separately or sequentially.

In another aspect, the invention provides for the use of a compound of formula (I) or a pharmaceutically acceptable salt thereof in the manufacture of a medicament for use in the treatment of the above disorders. In particular the present invention provides the use of a compound of formula (I) or a pharmaceutically acceptable salt thereof in the manufacture of a medicament for use in the treatment of a CNS disorder. Preferably the CNS disorder is depression or anxiety.

The composition of the present invention may contain from 0.1% to 99% by weight, preferably from 10 to 60% by weight, of the active material, depending on the method of administration. The dose of the compound used in the treatment of the aforementioned disorders will vary in the usual way with the seriousness of the disorders, the weight of the sufferer, and other similar factors. However, as a general guide suitable unit doses may be 0.05 to 1000 mg, more suitably 1.0 to 200 mg, and such unit doses may be administered more than once a day, for example two or three times a day. Such therapy may extend for a number of weeks or months. When administered in accordance with the invention, no unacceptable toxicological effects are expected with the compounds of the invention.

The following Descriptions and Examples illustrate the preparation of compounds of the present invention.

### Preparation 1: [4-Methoxy-3-(2-piperidin-1-yl-ethoxy)-phenyl]-acetic acid 2-piperidln-1-yl-ethyl ester

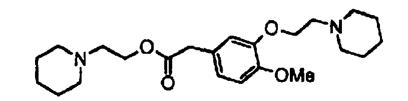

A mixture of (3-hydroxy-4-methoxy-phenyl)-acetic acid (1.85 g), dry DMF (25 ml), K₂CO₃ (5.9 g) and *N*-chloroethylpiperidine hydrochloride (3.74 g) was heated at 40°C for 5 h. Volatiles were then removed *in vacuo* and the residue partitioned between water and EtOAc. The organic layer was washed (brine) and concentrated to give the title (3.76 g) compound as an orange oil.
**NMR (¹H, CDCl₃):** δ 6.93-6.80 (m, 3H), 4.22 (t, 2H), 4.14 (t, 2H), 3.82 (s, 3H), 3.55 (s, 2H), 2.82 (t, 2H), 2.66-2.40 (m, 10H), 1.66-1.40 (m, 12H).

### Preparation 2: [4-Methoxy-3-(2-piperidin-1-yl-ethoxy)-phenyl]-acetic acid methyl ester

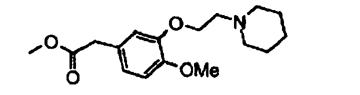

[4-Methoxy-3-(2-piperidin-1-yl-ethoxy)-phenyl]-acetic acid 2-piperidin-1-yl-ethyl ester (6.3 g) in MeOH (6 ml), THF (6 ml) and water (6 ml) containing KOH (1.7 g) was heated at 45 °C for 1 h and then allowed to cool to 25 °C over 90 min. With stirring in an ice bath conc. aqueous HCl (6 ml) was then added. The mixture was evaporated to dryness. The material was heated at reflux with HCl in MeOH (1 M) for 4 h, concentrated and extracted with CH₂Cl₂. The mixture was filtered and the solvent removed *in vacuo* to give the title compound (4.4 g) as an orange oil.
**NMR (¹H, CDCl₃)**: δ 6.88-6.79 (m, 3H), 4.18 (t, 2H), 3.84 (s, 3H), 3.69 (s, 3H), 3.55 (s, 2H), 2.87 (t, 2H), 2.56 (bs, 4H), 1.66 (bs, 4H), 1.46 (bs, 2H).

### Preparation 3: 2-[4-Methoxy-3-(2-piperidin-1-yl-ethoxy)-phenyl]-pent-4-enoic acid methyl ester

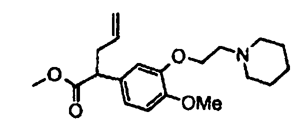

**Procedure**: To a solution of [4-methoxy-3-(2-piperidin-1-yl-ethoxy)-phenyl]-acetic acid methyl ester (2.1 g) in THF (dry, 15 ml) at -78°C was slowly added lithium bis(trimethylsilyl)amide (1 M in THF, 8.2 ml). The solution was stirred at this temperature for 15 min before allyl bromide (0.59 ml) was added. After additional 30 min water and EtOAc were added with stirring. The mixture was allowed to warm to 25°C, layers separated and the organic layer washed (brine), concentrated and submitted to column chromatography (silica gel, CH₂Cl₂ / MeOH / NH₃) to give the title compound (1.3 g) as a colourless oil.
**NMR (¹H, CDCl₃)**: δ 6.90-6.80 (m, 3H), 5.76-5.67 (m, 1H), 5.10-4.98 (m, 2H), 4.18 (bs, 2H), 3.83 (s, 3H), 3.65 (s, 3H), 3.56 (t, 1H), 2.86 (bs, 2H), 2.83-2.73 (m, 1H), 2.65-2.45 (m, 5H), 1.65 (bs, 4H), 1.47 (bs, 2H). **MS *(m*/*****z)**:* 348 [MH]⁺.

### Preparation 4: 2-[4-Methoxy-3-(2-piperidin-1-yl-ethoxy)-phenyl]-pent-4-enoic acid hydrochloride salt

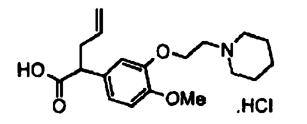

2-[4-Methoxy-3-(2-piperidin-1-yl-ethoxy)-phenyl]-pent-4-enoic acid methyl ester (1.3 g) in MeOH (2 ml), THF (2 ml) and water (2 ml) containing KOH (0.42 g) was heated at 45 °C for 1 h and then allowed to cool to 25°C. The mixture was evaporated to dryness. THF (5 ml) and conc. aqueous HCl (0.62 ml) were added, the mixture concentrated, extracted with CH₂Cl₂, filtered and the solvent removed *in vacuo* to give the title compound (1.2 g) as an off-white foam.
**NMR (¹H, CD₃OD):** δ 7.05 (d, 1H), 6.96 (dd, 1H), 6.91 (d, 1H), 5.81-5.70 (m, 1H), 5.02 (dd, 1H), 4.92 (dd, 1H), 4.31 (dd, 2H), 3.83 (s, 3H), 3.48-3.42 (m, 3H), 3.37-3.28 (m, 4H), 2.78-2.69 (m, 1H), 2.44-2.35 (m, 1H), 1.91-1.84 (m, 4H), 1.72-1.63 (m, 2H). **MS *(m*/*****z)**:* 334 [MH]⁺.

### Preparation 5: 2-[4-Methoxy-3-(2-piperidin-1-yl-ethoxy)-phenyl]-pent-4-enoic acid (3,4-dichlorophonyl)-amide

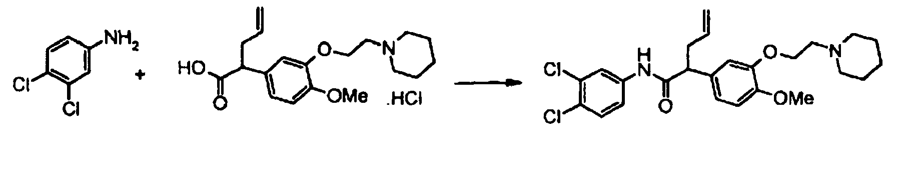

To a solution of 2-[4-methoxy-3-(2-piperidin-1-yl-ethoxy)-phenyl]-pent-4-enoic acid hydrochloride salt (0.46 g) in dry CH₂Cl₂ (4 ml), under N₂, was added, at 0°C, oxalyl chloride (0.11 ml) and DMF (cat). After 30 min the reaction mixture was concentrated to dryness *in vacuo.* To this material was added toluene (dry, 4 ml) and 3,4-dichloroaniline (0.20 g). The mixture was heated at 105 °C for 4 h, then partitioned between aqueous NaHCO₃ and EtOAc. The organic layer was washed (brine), concentrated and purified by column chromatography (silica gel, CH₂Cl₂ / MeOH NH₃) to give the title compound (0.35 g) as a slightly brown oil.
**NMR (¹H, CDCl₃):** δ 7.74 (s, 1H), 7.55 (bs, 1H), 7.31 (bs, 2H), 6.96 (bs, 1H), 6.91-6.83 (m, 2H), 5.77-5.68 (m, 1H), 5.09 (d, 1H), 5.01 (d, 1H), 4.23-4.16 (m, 2H), 3.85 (s, 3H), 3.53 (t, 1H), 2.99-2.91 (m, 1H), 2.83 (t, 2H), 2.55 (bs, 4H), 1.68-1.62 (m, 4H), 1.47 (bs, 3H). **MS (*m*/*z*)*:*** 477 [MH]⁺. 2Cl.

### Preparation 6: 2-[4-Methoxy-3-(2-piperidin-1-yl-ethoxy)-phenyl]-propanoic acid methyl ester

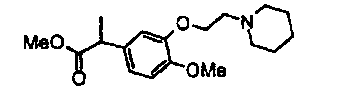

To a solution of [4-methoxy-3-(2-piperidin-1-yl-ethoxy)-phenyl]-acetic acid methyl ester (0.60 g) in THF (dry, 6 ml) at -78°C was slowly added lithium bis(trimethylsilyl)amide (1 M in THF, 2.3 ml). The solution was stirred at this temperature for 15 min before iodomethane (0.12 ml) was added, then allowed to warm to 25 °C. After 16 h aqueous NaHCO₃ and EtOAc were added with stirring, layers separated and the organic layer washed (brine), concentrated and submitted to column chromatography (silica gel, CH₂Cl₂ / MeOH / NH₃) to give the title compound (0.27 g) as a slightly yellow oil.
**NMR (¹H, CD₃OD)**: δ 6.88-6.75 (m, 3H), 4.12 (t, 2H), 3.80 (s, 3H), 3.67-3.34 (m, 4H), 2.78 (t, 2H), 2.50 (bs, 4H), 1.65-1.37 (m, 9H). **MS (m/z)**: 322 [MH]⁺.

### Preparation 7: 2-(3,4-Dichloro-phenylamino)-2-methyl-propionic acid

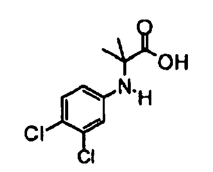

To a solution of 3,4-dichloroaniline (2 g, 12.4 mmol) and 1,1,1-trichloro-2-methyl-2-propanol 0.5 hydrate (3.47 g, 18.63 mmol) in acetone (25 ml) was added KOH (2.79 g, 49.7 mmol) at 0 °C, and the mixture was stirred for 1 h at 0 °C and overnight at room temperature. The reaction mixture was concentrated *in vacuo,* diluted with water, and washed with diethyl ether. The acqueous solution was acidified with citric acid and extracted with AcOEt (3x150 ml). The organic layer was washed with brine, dried over anhydrous NaSO₄ and concentrated *in vacuo* to give the title compound in 2.2 g yield as a white solid (72%).
**NMR (¹H, DMSO):** δ 12.30 (bs,1 H), 7.18 (d, 1H), 6.60 (s, 1H), 6.40 (dd, 1 H), 4.98 (bs, 1 H), 1.40 (s, 4H).

### Preparation 8: Methyl 2-(3,4-dichloro-phenylamino)-2-methyl-propionate

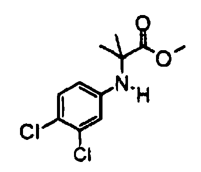

To a solution of 2-methyl-2-(3,4-dichloroaniline)-propionic acid (0.50 g, 2.02 mmol) in MeOH+CH₂Cl₂ (14+7 ml) were added triethylamine (0.57 ml, 4.08 mmol) and Me₃SiCHN₂ (2M in hexane, 6.06 mmol) at 0 °C. The reaction mixture was stirred for 4 h at room temperature, concentrated *in vacuo* and the crude product purified by flash chromatography (AcOEt:cyclohexane=1:9) to give the title compound in 489 mg yield as white solid (93%).
**NMR (¹H, DMSO):** δ 7.42 (dd,1H), 6.57 (d, 1H), 6.46 (s, 1H), 6.35 (dd, 1H), 3.62 (s, 3H), 1.45 (s, 6H).

### Preparation 9: 2-[4-Methoxy-3-(2-plperidin-1-yl-ethoxy)-phenyl]-acettc acid hydrochloride salt

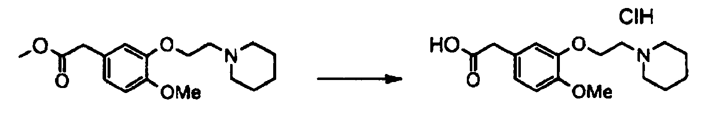

To a solution of methyl-2-(3-[2-piperidin-1-yl-ethoxy]-4-methoxy-phenyl)-acetate (0.59 g, 1.92 mmol) in THF+MeOH (1+1 ml) at 25°C was added a solution of KOH (0.21 g, 3.84 mmol) in H₂O (1 ml). The solution was heated at 45 °C for 1.5 hours. After removing the solvent *in vacuo* the residue was dissolved in THF and acidified at 0 °C with conc. aqueous HCl. The mixture was concentrated to dryness *in vacuo,* the crude was extracted with PrOH/CH₂Cl₂ (1/1), filtered and concentrated to dryness *in vacuo* to give the title compound in 544 mg yield as brown solid (y= 96%). **MS (*m*/*z*)*:*** 294 [MH]⁺.

### Preparation 10: 2-((3,4-Dichloro-phenyl)-{2-[4-methoxy-3-(2-piperidin-1-yl-ethoxy)-phenyl]-acetyl}-amino)-2-methyl propionic acid methyl ester

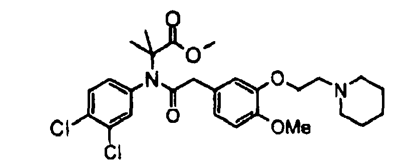

To a solution of 2-(3-[2-piperidin-1-yl-ethoxy]-4-methoxy-phenyl)-acetic acid hydrochloride salt (0.62 g, 1.89 mmol) in CH₂Cl₂ (9 ml) at 0 °C were added oxalyl chloride (0.33 ml, 3.78 mmol) and DMF (some drops). The solution was stirred at room temperature for 1.5 hours and concentrated to dryness *in vacuo*. The crude was dissolved in 1,4-dioxane, added with a solution of methyl-2-methyl-2-(3,4-dichloroaniline)-propionate (0.47 g, 1.78 mmol) in 1,4-dioxane and heated at 90 °C for 20 hours. The reaction mixture was concentrated *in vacuo*, dissolved in CH₂Cl₂, washed with conc. aqueous NaHCO₃, brine and dried over anhydrous NaSO₄. After purification by flash chromatography (CH₂Cl₂:MeOH=9:1) the title compound was obtained in 681 mg yield as a brown oil (61 %). **MS *(m*/*****z)**:* 537 [MH]⁺ (2Cl).

### Example 1: 1-(3,4-Dichloro-phenyl)-5-hydroxy-3-[4-methoxy-3-(2-piperidin-1-yl-ethoxy)-phenyl]-pyrrolidin-2-one

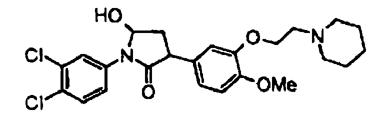

To a solution of 2-[4-methoxy-3-(2-piperidin-1-yl-ethoxy)-phenyl]-pent-4-enoic acid (3,4-dichlorophenyl)-amide (0.30 g) in THF/H₂O (5/1, 12 ml) was added OsO₄ (4% wt in water, 0.2 ml) and NalO₄ (0.41 g). After 20 hours excess aqueous Na₂S₂O₃ was added with stirring. After 5 min. the mixture was partitioned between sat. aqueous NaHCO₃ and EtOAc. The organic layer was washed (brine), concentrated to dryness *in vacuo* and the residue purified by column chromatography to give the title product as a colourless foam (0.20 g, ca. 2:1 mixture of diastereoisomers):
**NMR (¹H, CDCl₃):** δ 7.80 and 7.74 (2d, 1H), 7.55-7.35 (m, 2H), 7.00 (s, 0.33H), 6.92 (d, 0.33H), 6.83-6.70 (m, 2.34H), 5.70-5.59 (m, 1H), 4.17-4.00 (m, 3.67H), 3.82 (s, 3H), 3.70 (dd, 0.33H), 2.95-2.85 (m, 0.33H), 2.77 (t, 2H), 2.55-2.34 (m, 5.34H), 2.17-2.08 (m, 0.33H), 1.62-1.50 (m, 4H), 1.46-1.35 (m, 2H), **MS** ***(mlz)**:* 479 [MH]⁺, 2Cl.

### Example 2: 1-(3,4-Dichloro-phenyl)-3-[4-methoxy-3-(2-piperidin-1-yl-ethoxy)-phenyl]-1,6-dihydro-pyrrole-2-one

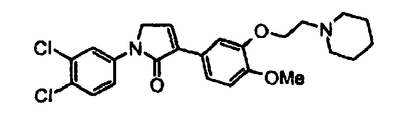

To 1-(3,4-dichloro-phenyl)-5-hydroxy-3-[4-methoxy-3-(2-piperidin-1-yl-ethoxy)-phenyl]-pyrrolidin-2-one (0.13 g) in CH₂Cl₂ (dry, 5 ml) and Et₃SiH (0.13 ml) at 0 °C was added dropwise a solution of trifluoroacetic acid (0.22 ml) in CH₂Cl₂ (dry, 1 ml). The mixture was allowed to warm to 25 °C. After 20 h volatiles were removed *in vacuo* and the residue submitted to column chromatography (silica gel, CH₂Cl₂ / MeOH / NH₃) to give a mixture of 1 -(3,4-dichloro-phenyl)-3-[4-methoxy-3-(2-piperidin-1-yl-ethoxy)-pheny]-pyrrolidin-2-one and 1-(3,4-dichloro-phenyl)-3-[4-methoxy-3-(2-piperidin-1-yl-ethoxy)-phenyl]-1,5-dihydro-pyrrole-2-one. On trituration with EtOAc : petroleum ether (40-60) 1:2 the title compound was obtained as an off-white solid.
**NMR (¹H, CDCl₃)**: δ 7.99 (d, 1H), 7.70 (dd, 1H), 7.54-7.50 (m, 2H), 7.46 (d, 1H), 7.22 (t, 1H), 6.92 (d, 1H), 4.43 (d, 2H), 4.23 (t, 2H), 3.91 (s, 3H), 2.87 (t, 2H), 2.56 (bs, 4H), 1.70-1.60 (m, 4H), 1.47 (bs, 2H). MS (*m*/*z*)*:* 461 [MH]⁺, 2Cl. mp : 98-99°C.

### Example 3: 1-(3,4-Dichloro-phenyl),3-[4-methoxy-3-(2-piperidin-1-yl-ethoxy)-phenyl]-pyrrolidin-2-one

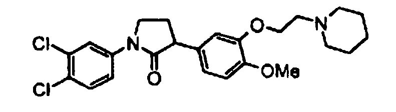

The title compound (22 mg) was obtained as a colourless film from the mother liquor from Example 2 by removal of the volatiles, extraction with hexane and concentration of the hexane solubles *in vacuo.*
**NMR (¹H, CDCl₃)**: δ 7.87 (d, 1H), 7.60 (dd, 1H), 7.43 (d, 1H), 6.89-6.83 (m, 3H), 4.17 (t, 2H), 3.95-3.80 (m, 6H), 2.82 (t, 2H), 2.70-2.60 (m, 1H), 2.52 (bs, 4H), 2.35-2.25 (m, 1H), 1.64-1.58 (m, 4H), 1.50-1.42 (m, 2H). **MS *(m*/*****z)**:* 463 [MH]⁺, 2Cl.

### Example 4: 1-(3,4-Dichloro-phenyl)-5-methoxy-3-[4-methoxy-3-(2-piperidin-1-ylethoxy)-phenyl]-pyrrolidin-2-one

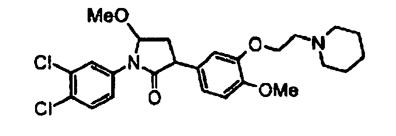

1-(3,4-Dichloro-phenyl)-5-hydroxy-3-[4-methoxy-3-(2-piperidin-1-yl-ethoxy)-phenyl]-pyrrolidin-2-one was treated with MeOH (dry, 2 ml) containing pyridinium 4-toluenesulfonate (42 mg) at 25 °C for 4 days, at 50 °C for 4 h and at 60°C for 6 h. The mixture was partitioned between aqueous NaHCO₃ and EtOAc. The organic layer was collected, concentrated and submitted to column chromatography (silica gel, CH₂Cl₂ / MeOH / NH₃). A major diastereoisomer (3,5*-trans* substitution of the pyrrolidin-2-one, 25 mg slightly yellow film) was isolated besides a *ca.* 1:1 mixture of the major and the minor diastereoisomer (4 mg colourless film) and recovered unreacted starting material (21 mg 1-(3,4-dichloro-phenyl)-5-hydroxy-3-[4-methoxy-3-(2-piperidin-1-yl-ethoxy)-phenyl]-pyrrolidin-2-one).
**Major diastereoisomer: NMR (¹H, CDCl₃)**: δ 7.81 (d, 1H), 7.51 (d, 1H), 7.45 (d, 1H), 6.90-6.82 (m, 3H), 5.32 (d, 1H), 4.17 (t, 2H), 4.02 (dd, 1H), 3.85 (s, 3H), 3.41 (s, 3H), 2.84 (t, 2H), 2.64 (dd, 1H), 2.56 (bs, 4H), 2.37-2.30 (m, 1H), 1.67-1.60 (m, 4H), 1.50-1.40 (m, 2H). **MS *(m*/*****z)**:* 493 [MH]⁺, 2Cl.
**Ca. 1:1 mixture of major and minor diastereoisomer: NMR (¹H, CDCl₃):** δ 7.82 and 7.74 (2d, 1H), 7.51 (d, 0.5H), 7.48-7.42 (m, 1H), 7.03 (d, 0.5H), 6.97 (dd, 0.5 H), 6.91-6.83 (m, 2.5H), 5.37-5.33 (m, 1H), 4.26-4.20 (m, 2H), 4.02 (dd, 0.5H), 3.85 (2s, 3H), 3.77 (dd, 0.5H), 3.41 and 3.38 (2s, 3H), 2.89 (bs, 2H), 2.85-2.78 (m, 0.5H), 2.70-2.50 (m, 4.5H), 2.37-2.24 (m, 1H), 1.66 (bs, 4H), 1.48 (bs, 2H). **MS** *(mlz):* 493 [MH]⁺, 2Cl.

### Example 5: 1-(4-Methoxyphenyl)-3-[4-methoxy-3-(2-piperidin-1-yl-ethoxy)-phenyl]-1,5-dihydro-pyrrole-2-one hydrochloride

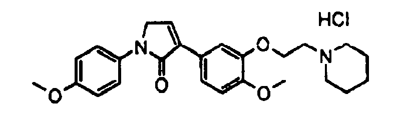

To a solution of 2-[4-methoxy-3-(2-piperidin-1-yl-ethoxy)-phenyl]-pent-4-enoic acid (4-methoxyphenyl)-amide (prepared in an analogous way to Procedure 5, 570 mg, 1.3 mmol) in acetone/H₂O 8/1 (33 ml) was added N-methyl-morpholine-N-oxide (2eq, 304 mg) and OsO₄ (4wt% sol. in water, cat., 0.84 ml). The reaction was stirred at room temperature for 6 hours and then quenched with 40 ml of Na₂SO₃ sat. After 15 minutes stirring the diol was extracted with ethyl acetate (2x20 ml), dried over Na₂SO₄, filtered and concentrated to dryness *in vacuo.*
The crude product was then dissolved in THF/H₂O 1/1 (30 ml) and potassium periodate (1.5 eq, 391 mg) was added. The reaction mixture was stirred at room temperature for 3 hours. The solution was diluted with water (20 ml) and extracted with ethyl acetate (3x20 ml). The combined organic extracts were dried over anhydrous Na₂SO₄, filtered and concentrated to dryness *in vacuo.* Flash chromatography of the crude product (silica gel, CH₂Cl₂/MeOH/NH₃ aq. 150/10/1) gave 245 mg of the cyclized product.
This material (130 mg) was dissolved in TFA (2.5 ml). The reaction mixture was stirred at room temperature for 2 hours, then concentrated *in vacuo.* A saturated solution of NaHCO₃ was added and the mixture was extracted with ethyl acetate, dried over Na₂SO₄ and concentrated *in vacuo.* The residue was dissolved in CH₂Cl₂ and HCl (1M in Et₂O, 2 ml) was added, the volatiles evaporated and the residue triturated with Et₂O to give 130 mg of the title product as a light pink solid:
**NMR (¹H, DMSO-d6):** δ 9.9 (bs 1H), 7.71 (d, 2H), 7.7-6.64 (m, 2H), 7.67 (t, 1H), 7.09 (d, 1H), 6.99 (d, 2H), 4.56 (d, 2H), 4.39 (bt, 2H), 3.81 (s, 3H), 3.76 (s, 3H), 3.58 (m, 2H), 3.50 (m, 2H), 3.04 (m, 2H), 1.81 (m, 4H), 1.7 (m, 1H), 1.4 (m, 1H). **MS *(m*/*****z):*** 423 [MH]⁺.

### Example 6: 1-(4-Methoxyphenyl)-3-[4-methoxy-3-(2-piperidin-1-yl-ethoxy)-phenyl]-pyrrolidin-2-one hydrochloride

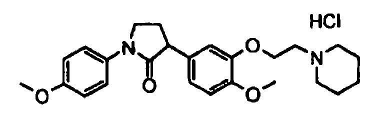

The product was prepared in an analogous way to the one described for Example 3.
**NMR (¹H, DMSO-d6):** δ 9.87 (bs, 1H), 7.58 (d, 2H), 7.0-6.8 (m, 5H), 4.33 (t, 2H), 3.85 (m, 2H), 3.84 (t, 1H), 3.77 (s, 3H), 3.74 (s, 3H), 3.55 (d, 2H) 3.45 (m, 2H), 3.02 (m, 2H), 2.53 (m, 1H), 2.16 (m, 1H), 1.85-1.65 (m, 5H), 1.39 (m, 1 H). **MS (*m*/*z*)*:*** 425 [MH]⁺.

### Example 7: 1-(3-Chloro-4-methoxy-phenyl)-3-[4-methoxy-3-(2-piperidin-1-yl-ethoxy)-phenyn-pyrrolidin-2-one hydrochloride

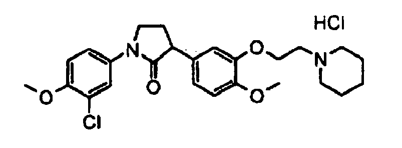

The product was prepared in an analogous way to the one described for Example 3.
**NMR (¹H, DMSO-d6):** δ 9.75 (bs, 1H), 7.89 (d, 1 H), 7.53 (dd, 1H), 7.17 (d, 1H), 6.99 (m, 2H), 6.90 (dd, 1H), 4.32 (t, 2H), 3.87 (m, 3H), 3.83 (s, 3H), 3.81 (s, 3H), 3.55 (m, 2H), 3.45 (m, 2H), 3.0 (m, 2H), 2.5 (m, 1 H), 2.15 (m, 1 H), 1.9 (m, 4H), 1.4 (m, 2H). **MS *(m*/*****z)**:* 459 [MH]⁺, 1Cl.

### Example 8: 1-(3-Chloro-4-methoxy-phenyl)-3-[4-methoxy-3-(2-piperidin-1-yl-ethoxy)-phenyl]-1,5-dihydro-pyrrole-2-one hydrochloride

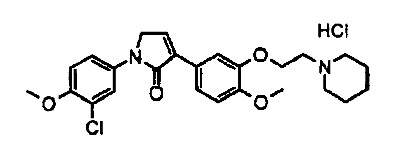

The product was prepared in an analogous way to the one described for Example 6.
**NMR (¹H, DMSO-d6):** δ 9.76 (bs, 1H), 8.01 (d, 1H), 7.68 (m, 4H), 7.19 (d, 1H), 7.08 (d, 1H), 4.59 (d, 2H), 4.38 (t, 2H), 3.85 (s, 3H), 3.81 (s, 3H), 3.58 (m, 2H), 3.50 (m, 2H), 3.04 (m, 2H), 1.85 (m, 4H), 1.4 (m, 2H). **MS *(m*/*****z)**:* 457 [MH]⁺, 1CI.

### Example 9: 1-(3-Fluoro-4-methoxy-phenyl)-3-[4-methoxy-3-(2-piperidin-1-yl-ethoxy)-phenyl]-pyrrolidin-2-one hydrochloride

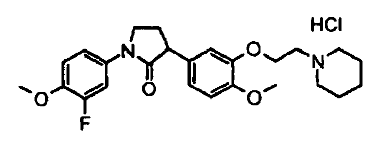

The product was prepared in an analogous way to the one described for Example 3.
**NMR (¹H, DMSO-d6)**: δ 9.82 (bs, 1H), 7.74 (dd, 1H), 7.36 (dd, 1H), 7.17 (t, 1H), 6.99 (m, 2H), 6.90 (dd, 1 H), 4.32 (t, 2H), 3.87 (m, 3H). 3.82 (s, 3H), 3.76 (s, 3H), 3.54 (m, 2H), 3.45 (m, 2H), 3.0 (m, 2H), 2.5 (m, 1H), 2.16 (m, 1H), 1.85 (m, 4H), 1.4 (m, 2H). **MS *(m*/*****z)**: 443* [MH]⁺.

### Example 10: 1-(3-Fluoro-4-methoxy-phenyl)-3-[4-methoxy-3-(2-piperidin-1-yl-ethoxy)-phenyl]-1,5-dihydro-pyrrole-2-one hydrochloride

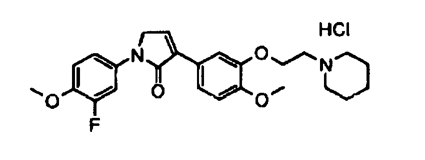

The product was prepared in an analogous way to the one described for Example 6.
**NMR (¹H, DMSO-d6):** δ 9.87 (bs, 1H), 7.84 (dd, 1H), 7.68 (m, 2H), 7.66 (dd, 1H), 7.51 (m, 1H), 7.21 (t, 1H), 7.08 (d, 1H), 4.58 (d, 2H), 4.39 (t, 2H), 3.84 (s, 3H), 3.81 (s, 3H), 3.57 (bd, 2H), 3.49 (m, 2H), 3.04 (m, 2H), 1.9-1.75 (m, 5H), 1.4 (m, 1 H). **MS *(m*/*****z)**:* 441 [MH]⁺.

### Example 11: 1-(3-Fluoro-phenyl)-3-[4-methoxy-3-(2-pipsridin-1-yl-ethoxy)-phenyl]-pyrrolidin-2-one hydrochloride

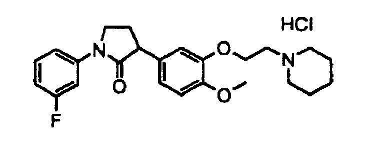

The product was prepared in an analogous way to the one described for Example 3.
**NMR (¹H, DMSO-d6):** δ 9.75 (bs, 1H), 7.70 (m, 1H), 7.47 (m, 1H), 7.43 (m, 1H), 7.0 (m, 1H), 7.0 (m, 2H), 6.92 (dd, 1H), 4.32 (t, 2H), 3.90 (m, 3H), 3.77 (s, 3H), 3.54 (m, 2H), 3.46 (m, 2H), 3.0 (m, 2H), 2.5-2.2 (m, 2H), 1.82 (m, 4H), 1.4 (m, 2H). **MS** *(m*/*z):* 413 [MH]⁺.

### Example 12: 1-(3-Fluoro-phenyl)-3-[4-methoxy-3-(2-piperidin-1-yl-ethoxy)-phenyl]-1,5-dihydro-pyrrole-2-one hydrochloride

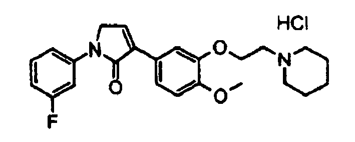

The product was prepared in an analogous way to the one described for Example 6.
**NMR (¹H, DMSO-d6):** δ 9.9 (bs, 1H), 7.82 (m, 1H), 7.73 (t, 1 H), 7.66 (m, 2H), 7.60 (dd, 1H), 7.45 (m, 1H), 7.09 (d, 1H), 6.97 (m, 1H), 4.62 (d, 2H), 4.39 (t, 2H), 3.82 (s, 3H), 3.57 (m, 2H), 3.48 (m, 2H), 3.02 (m, 2H), 1.9 (m, 4H), 1.4 (m, 2H). **MS *(m*/*****z)**:* 411 [MH]⁺.

### Example 13: 1-(3,4-Dichloro-phenyl)-5,5-dimethyl-4-hydroxy-3-[4-methoxy-3-(2-piperidin-1-yl-ethoxy)-phenyl]-pyrrol-2-one

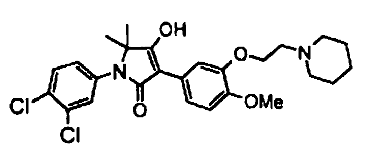

To a solution of 2-((3,4-dichloro-phenyl)-{2-[4-methoxy-3-(2-piperidin-1-yl-ethoxy)-phenyl]-acetyl}-amino)-2-methyl propionic acid methyl ester (0.19 g, 0.35 mmol) in CH₂Cl₂+THF (1.5+1.5 ml) at 0 °C was added NaH (60% in mineral oil, 13.9 mg, 0.35 mmol). The solution was stirred at room temperature for 1.5 hours, cooled at 0 °C and HCl (1M in Et₂O, 0.35 mmol) was added. After stirring the solution at room temperature for 15 minutes, the solvent was removed *in vacuo,* the crude was dissolved in CH₂Cl₂, filtered and concentrated to dryness *in vacuo.* After purification by flash chromatography CH₂Cl₂:MeOH=(9:1) the title compound was obtained in 143 mg yield as a yellow solid (82%).
**NMR (¹H, DMSO):** δ 9.50 (bs, 1H), 8.16 (bs, 1H), 7.97 (bd, 1 H), 7.83 (d, 1 H), 7.53 (d, 1H), 7.34 (dd, 1H), 6.87 (d, 1 H), 4.21 (t, 2H), 3.83 (t, 2H), 3.69 (s, 3H), 3.27 (m, 4H), 1.71 (m, 4H), 1.50 (bm, 2H), 1.25 (s, 6H). **MS *(m*/*****z)**:* 505 [MH]⁺ (2Cl).

### Example 14: 1-(3,4-Dichloro-phenyl)-5,5-dimethyl-4-hydroxy-3-[4-methoxy-3-(2-piperidin-1-yl-ethoxy)-phenyl]-pyrrol-2-one hydrochloride salt

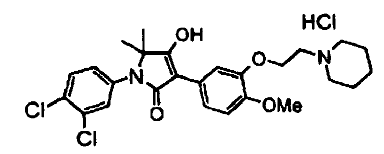

To a solution of 1-(3,4-dichloro-phenyl)-5,5-dimethyl-4-hydroxy-3-[4-methoxy-3-(2-piperidin-1-yt-ethoxy)-phenyl]-pyrrol-2-one in CH₂Cl₂ was added excess HCl (1M in Et₂O). The resulting mixture was evaporated to dryness and the residue triturated with Et₂O and dried to give the title compound in 5 mg yield as a white solid (66%).

## Claims

1. A compound of formula (I) or a pharmaceutically acceptable salt thereof:
wherein: 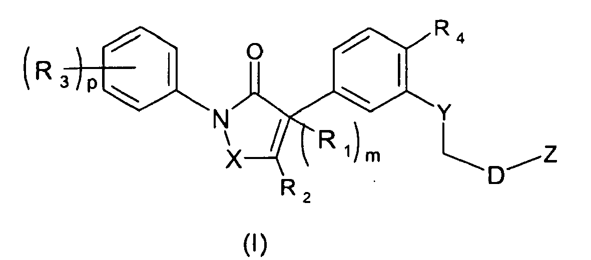
R₁ is hydrogen;
m is 0 when = is a double bond and m is 1 when = is a single bond;
R₂ is hydrogen, C₁₋₆alkyl;
X is a group -(CHR₅)- wherein R₅ is hydrogen, C₁₋₆alkoxy;
R₃ is halogen, C₁₋₆alkoxy;
p is 1 or 2;
R₄ is methoxy;
Y is oxygen;
D is -CH₂-; and
Z is is pyperidyl.

2. A compound as claimed in claim 1, wherein when = is a single bond.

3. A compound as claimed in claim 1 having the following formula (Ia): 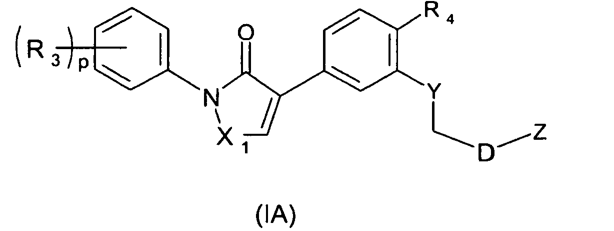 wherein R₃, p, R₄, Y, D, Z, = are as defined in claim 1 or 2 and X₁ is -CH₂- or -HC(OH)-.

4. A compound as claimed in any of claims 1-3, wherein p is 1 or 2 and R₃ is/are halogen attached at the 3 or the 3,4-positions of the phenyl ring.

5. A compound as claimed in claim 4, wherein halogen is chloro or fluoro.

6. A compound as claimed in claim 1 which is:
1-(3,4-Dichloro-phenyl)-5-hydroxy-3-[4-methoxy-3-(2-piperidin-1-yl-ethoxy)-phenyl]-pyrrolidin-2-one
1-(3,4-Dichloro-phenyl)-3-[4-methoxy-3-(2-piperidin-1-yl-ethoxy)-phenyl]-1,5-dihydro-pyrrole-2-one
1-(3,4-Dichloro-phenyl)-3-[4-methoxy-3-(2-piperidin-1-yl-ethoxy)-phenyl]-pyrrolidin-2-one
1-(3,4-Dichloro-phenyl)-5-methoxy-3-[4-methoxy-3-(2-piperidin-1-yl-ethoxy)-phenyl]-pyrrolidin-2-one
1-(4-Methoxyphenyl)-3-[4-methoxy-3-(2-piperidin-1-yl-ethoxy)-phenyl]-1,5-dihydro-pyrrole-2-one
1-(4-Methoxyphenyl)-3-[4-methoxy-3-(2-piperidin-1-yl-ethoxy)-phenyl]-pyrrolidin-2-one
1-(3-Chloro-4-methoxy-phenyl)-3-[4-methoxy-3-(2-piperidin-1-yl-ethoxy)-phenyl]-pyrrolidin-2-one
1-(3-Chloro-4-methoxy-phenyl)-3-[4-methoxy-3-(2-piperidin-1-yl-ethoxy)-phenyl]-1,5-dihydro-pyrrole-2-one
1-(3-Fluoro-4-methoxy-phenyl)-3-[4-methoxy-3-(2-piperidin-1-yl-ethoxy)-phenyl]-pyrrolidin-2-one
1-(3-Fluoro-4-methoxy-phenyl)-3-[4-methoxy-3-(2-piperidin-1-yl-ethoxy)-phenyl]-1,5-dihydro-pyrrole-2-one
1-(3-Fluoro-phenyl)-3-[4-methoxy-3-(2-piperidin-1-yl-ethoxy)-phenyl]-pyrrolidin-2-one
1-(3-Fluoro-phenyl)-3-[4-methoxy-3-(2-piperidin-1-yl-ethoxy)-phenyl]-1,5-dihydro-pyrrole-2-one
1-(3,4-Dichloro-phenyl)-5,5-dimethyl-4-hydroxy-3-[4-methoxy-3-(2-piperidin- I -yl-ethoxy)-phenyl]-pyrrol-2-one or a pharmaceutically acceptable salt thereof.

7. A process for the preparation of a compound as defined in any of claims 1-6, which process comprises:
(a) reacting a compound of formula (II): 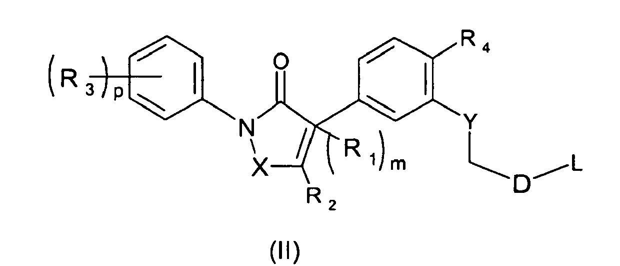 wherein R₁, R₂, R₃, R₄, m, p, X, =, Y and D are as defined for formula (I), and L is a leaving group, with a compound of formula (III):
Z-H (III)
wherein Z is as defined for formula (I); or 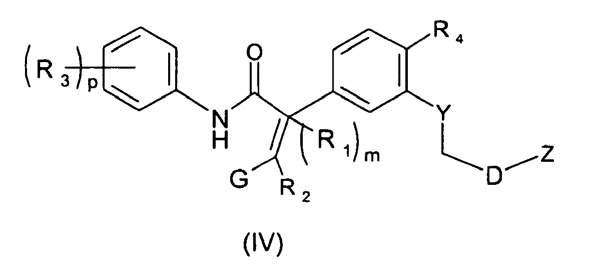
(b) cyclising a compound of formula (IV):
wherein R₁, R₂, m, R₃, p, R4, Y, D, Z and = are as defined for formula (I) and G is a group - X=CH₂, wherein X is as defined for formula (I), dehydrogenated as required;
and thereafter, for either process (a) or process (b), optionally followed by:
• removing any protecting groups; and/or
• converting a compound of formula (I) into another compound of formula (I); and/or
• forming a pharmaceutically acceptable salt.

8. A pharmaceutical composition comprising a compound as defined in any of claims 1-6 and a pharmaceutically acceptable carrier or excipient.

9. A process for preparing a pharmaceutical composition as defined in claim 7, the process comprising mixing a compound as defined in any of claims 1-6 and a pharmaceutically acceptable carrier or excipient.

10. A compound as defined in any of claims 1-6 for use as a therapeutic substance.

11. A compound as defined in any of claims 1-6 for use in the treatment of a CNS disorder.

12. A compound as defined in any of claims 1-6 for use in the treatment of depression or anxiety.

13. Use of a compound as defined in any of claims 1-6 in the manufacture of a medicament for use in the treatment of a CNS disorder.

14. The use of a compound as defined in any of claims 1-6 in the manufacture of a medicament for use in the treatment of depression or anxiety.

## Patentansprüche

1. Verbindung der Formel (I) oder ein pharmazeutisch verträgliches Salz davon, wobei: 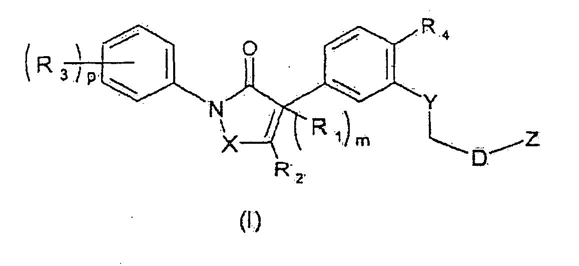
R₁ Wasserstoff ist;
m gleich 0 ist, wenn = eine Doppelbindung ist und m gleich 1 ist, wenn = eine Einfachbindung ist;
R₂ Wasserstoff oder C₁₋₆-Alkyl ist;
X ein Rest -(CHR₅)- ist, wobei R₅ Wasserstoff oder C₁₋₆-Alkoxy ist;
R₃ Halogen oder C₁₋₆-Alkoxy ist;
p gleich 1 oder 2 ist;
R₄ Methoxy ist;
Y Sauerstoff ist;
D -CH₂- ist; und
Z Piperidyl ist.

2. Verbindung nach Anspruch 1, wobei = eine Einzelbindung ist.

3. Verbindung nach Anspruch 1 mit der folgenden Formel (Ia): 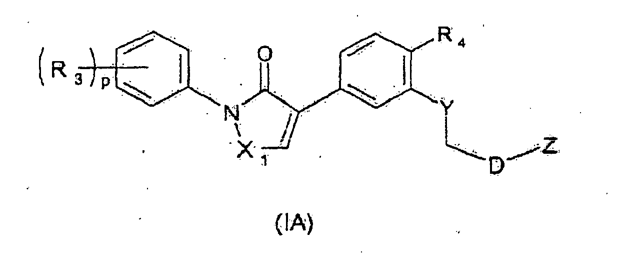 wobei R₃, p, R₄, Y, D, Z, = wie in Anspruch 1 oder 2 definiert sind und X₁ für -CH₂-oder -HC(OH)- steht.

4. Verbindung nach einem der Ansprüche 1 bis 3, wobei p gleich 1 oder 2 ist und R₃ Halogen ist/sind, gebunden an die 3- oder die 3,4-Positionen des Phenylrings.

5. Verbindung nach Anspruch 4, wobei Halogen Chlor oder Fluor ist.

6. Verbindung nach Anspruch 1, nämlich:
1-(3,4-Dichlorphenyl)-5-hydroxy-3-[4-methoxy-3-(2-piperidin-1-ylethoxy)-phenyl]-pyrrolidin-2-on;
1-(3,4-Dichlorphenyl)-3-[4-methoxy-3-(2-piperidin-1-ylethoxy)-phenyl]-1,5-dihydropyrrol-2-on;
1-(3,4-Dichlorphenyl)-3-[4-methoxy-3-(2-piperidin-1-ylethoxy)-phenyl]-pyrrolidin-2-on;
1-(3,4-Dichlorphenyl)-5-methoxy-3-[4-methoxy-3-(2-piperidin-1-ylethoxy)-phenyl]-pyrrolidin-2-on;
1-(4-Methoxyphenyl)-3-[4-methoxy-3-(2-piperidin-1-ylethoxy)-phenyl]-1,5-dihydropyrrol-2-on;
1-(4-Methoxyphenyl)-3-[4-methoxy-3-(2-piperidin-1-ylethoxy)-phenyl]-pyrrolidin-2-on;
1-(3-Chlor-4-methoxyphenyl)-3-[4-methoxy-3-(2-piperidin-1-ylethoxy)-phenyl]-pyrrolidin-2-on;
1-(3-Chlor-4-methoxyphenyl)-3-[4-methoxy-3-(2-piperidin-1-ylethoxy)-phenyl]-1,5-dihydropyrrol-2-on;
1-(3-Fluor-4-methoxyphenyl)-3-[4-methoxy-3-(2-piperidin-1-ylethoxy)-phenyl]-pyrrolidin-2-on;
1-(3-Fluor-4-methoxyphenyl)-3-[4-methoxy-3-(2-piperidin-1-ylethoxy)-phenyl]-1,5-dihydropyrrol-2-on;
1-(3-Fluorphenyl)-3-[4-methoxy-3-(2-piperidin-1-ylethoxy)-phenyl]-pyrrolidin-2-on;
1-(3-Fluorphenyl)-3-[4-methoxy-3-(2-piperidin-1-ylethoxy)-phenyl]-1,5-dihydropyrrol-2-on;
1-(3,4-Dichlorphenyl)-5,5-dimethyl-4-hydroxy-3-[4-methoxy-3-(2-piperidin-1-ylethoxy)-phenyl]-pyrrol-2-on oder ein pharmazeutisch verträgliches Salz davon.

7. Verfahren zur Herstellung einer Verbindung nach einem der Ansprüche 1 bis 6, wobei das Verfahren umfasst:
(a) Umsetzen einer Verbindung der Formel (II): 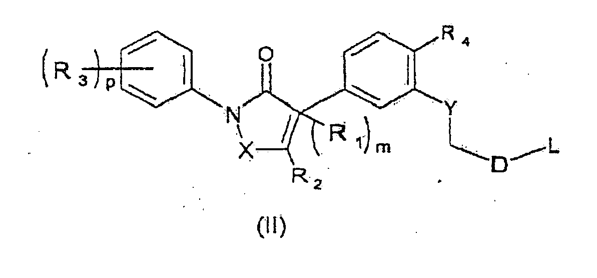 wobei R₁, R₂, R₃, R₄, m, p, X, =, Y und D wie für Formel (I) definiert sind und L eine Abgangsgruppe ist, mit einer Verbindung der Formel (III):
Z-H (III)
wobei Z wie für Formel (I) definiert ist; oder 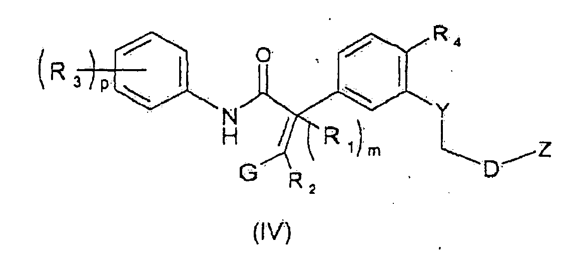
(b) Ringschluss einer Verbindung der Formel (IV):
wobei R₁, R₂, m, R₃, p, R₄, Y, D, Z und = wie für Formel (I) definiert sind und G ein Rest -X=CH₂ ist, wobei X wie für Formel (I) definiert ist, nach Bedarf dehydrogeniert;
und anschließend, entweder für Verfahren (a) oder Verfahren (b), gegebenenfalls gefolgt von:
• Entfernen jeglicher Schutzgruppen; und/oder
• Umwandeln einer Verbindung der Formel (I) in eine andere Verbindung der Formel (I); und/oder .
• Bilden eines pharmazeutisch verträglichen Salzes.

8. Arzneimittel, umfassend eine Verbindung nach einem der Ansprüche 1 bis 6 und einen pharmazeutisch verträglichen Träger oder Hilfsstoff.

9. Verfahren zur Herstellung eines Arzneimittels nach Anspruch 7, wobei das Verfahren Mischen einer Verbindung wie in einem der Ansprüche 1 bis 6 definiert und eines pharmazeutisch verträglichen Trägers oder Hilfsstoffes umfasst.

10. Verbindung nach einem der Ansprüche 1 bis 6 zur Verwendung als Arzneistoff.

11. Verbindung nach einem der Ansprüche 1 bis 6 zur Verwendung bei der Behandlung einer ZNS Störung.

12. Verbindung nach einem der Ansprüche 1 bis 6 zur Verwendung bei der Behandlung von Depression oder Angst.

13. Verwendung einer Verbindung nach einem der Ansprüche 1 bis 6 zur Herstellung eines Medikaments zur Verwendung bei der Behandlung einer ZNS Störung.

14. Verwendung einer Verbindung nach einem der Ansprüche 1 bis 6 zur Herstellung eines Medikaments zur Verwendung bei der Behandlung von Depression oder Angst.

## Revendications

1. Composé de formule (I) ou un de ses sels pharmaceutiquement acceptables : 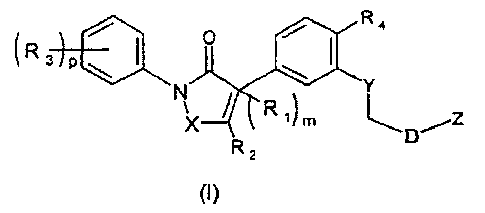 formule dans laquelle :
R₁ représente un atome d'hydrogène ;
m est égal à 0 lorsque = représente une double liaison et m est égal à 1 lorsque = représente une li aison simple ;
R₂ représente un atome d'hydrogène ou un groupe alkyle en C₁ à C₆ ;
X représente un groupe - (CHR₅) - dans lequel R₅ représente un atome d'hydrogène ou un groupe alkoxy en C₁ à C₆ ;
R₃ représente un atome d'halogène ou un groupe alkoxy en C₁ à C₆ ;
p est égal à 1 ou 2 ;
R₄ représente un groupe méthoxy ;
Y représente un atome d'oxygène ;
D représente un groupe -CH₂- ; et
Z représente un groupe pipéridyle.

2. Composé suivant la revendication 1, dans lequel = représente une liaison simple.

3. Composé suivant la revendication 1, répondant à la formule (Ia) suivante : 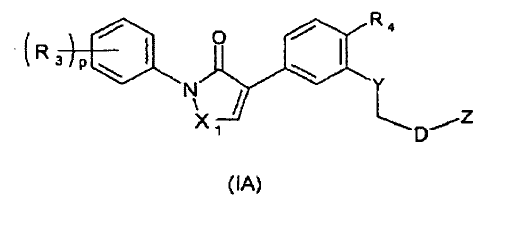 dans laquelle R₃, p, R₄, Y, D, Z, et = sont tels que définis dans la revendication 1 ou 2 et X ₁ représente un groupe -CH₂- ou -HC (OH) - .

4. Composé suivant l'une quelconque des revendications 1 à 3, dans lequel p est égal à 1 ou 2 et le ou les groupes R₃ représentent un atome d'halogène fixé en position 3 ou en positions 3,4 du noyau phényle.

5. Composé suivant la revendication 4, dans lequel le groupe halogéno est un groupe chloro ou fluoro.

6. Composé suivant la revendication 1, qui est
la 1-(3,4-dichloro-phényl)-5-hydroxy-3-[4-méthoxy-5-(2-pipéridine-1-yl-éthoxy)-phényl]-pyrrolidine-2-one
la 1-(3,4-dichloro-phényl)-3-[4-méthoxy-3-(2-pipéridin-1-yl-éthoxy)-phényl]-1,5-dihydro-pyrrole-2-one
la 1-(3,4-dichloro-phényl)-3-[4-méthoxy-3-(2-pipéridine 1-yl-éthoxy)-phényl]-pyrrolidine-2-one
la 1-(3,4-dichloro-phényl)-5-méthoxy-3-[4-méthoxy-3-(2-pipéridine-1-yl-éthoxy)-phényl]-pyrrolidine-2-one
la 1-(4-méthoxyphényl)-3-[4-méthoxy-3-(2-pipéridine-1-yl-éthoxy)-phényl]-1,5-dihydro-pyrrole-2-one
la 1-(4-méthoxyphényl)-3-[4-méthoxy-3-(2-pipéridine-1-yl-éthoxy)-phényl]-pyrrolidine-2-one
la 1-(3-chloro-4-méthoxyphényl)-3-[4-méthoxy-3-(2-pipéridine 1-yl-éthoxy)-phényl]-pyrrolidine-2-one
la 1-(3-chloro-4-méthoxyphényl)-3-[4-méthoxy-3-(2-pipéridine 1-yl-éthoxy)-phényl]-1,5-dihydro-pyrrole-2-one
la 1-(3-fluoro-4-méthoxyphényl)-3-[4-méthoxy-3-(2-pipéridine 1-yl-éthoxy)-phényl]-pyrrolidine-2-one
la 1-(3-fluoro-4-méthoxyphényl)-3-[4-méthoxy-3-(2-pipéridine 1-yl-éthoxy)-phényl]-1,5-dihydro-pyrrole-2-one
la 1-(3-fluorophényl)-3- [4-méthoxy-3- (2-pipéridine-1-yl-éthoxy)-phényl]-pyrrolidine-2-one
la 1-(3-fluorophényl)-3- [4-méthoxy-3-(2-pipéridine-1-yl-éthoxy)-phényl]-1,5-dihydro-pyrrole-2-one
la 1-(3,4-dichloro-phényl)-5,5-diméthyl-4-hydroxy-3-[4-méthoxy-3-(2-pipéridine-1-yl-éthoxy)-phényl]-pyrrole-2-one, ou un de ses sels pharmaceutiquement acceptables.

7. Procédé pour la préparation d'un composé suivant l'une quelconque des revendications 1 à 5, procédé qui comprend :
(a) la réaction d'un composé de formule (II) : 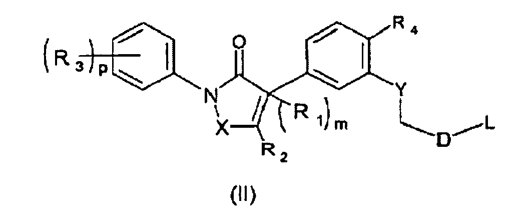 dans laquelle R₁, R₂, R₃, R₄, m, p, X, =, Y et D sont tels que définis pour la formule (I), et L représente un groupe partant, avec un composé de formule (III) :
Z-H (III)
dans laquelle Z est tel que défini pour la formule (I) ; ou
(b) la cyclisation d'un composé de formule (IV) : 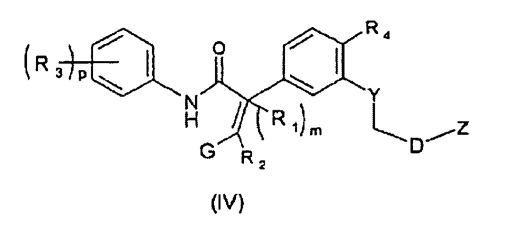
dans laquelle R₁, R₂, m, R₃, p, R₄, Y, D, Z et = sont tels que définis pour la formule (I) et G représente un groupe -X=CH₂, dans lequel X est tel que défini pour la formule (I), déshydrogéné de la manière requise ;
et ensuite, pour soit le procédé (a) soit le procédé (b), facultativement :
• l'élimination de tous groupes protecteurs ; et/ou
• la conversion d'un composé de formule (I) en un autre composé de formule (I) ; et/ou
• la formation d'un sel pharmaceutiquement acceptable.

8. Composition pharmaceutique comprenant un composé suivant l'une quelconque des revendications 1 à 6 et un support ou excipient pharmaceutiquement acceptable.

9. Procédé pour la préparation d'une composition pharmaceutique suivant la revendication 7, comprenant le mélange d'un composé suivant l'une quelconque des revendications 1 à 6, et d'un support ou excipient pharmaceutiquement acceptable.

10. Composé suivant l'une quelconque des revendications 1 à 6, destiné à être utilisé comme substance thérapeutique.

11. Composé suivant l'une quelconque des revendications 1 à 6, destiné à être utilisé dans le traitement d'un trouble du SNC.

12. Composé suivant l'une quelconque des revendications 1 à 6, destiné à être utilisé dans le traitement de la dépression ou de l'anxiété.

13. Composé suivant l'une quelconque des revendications 1 à 6, dans la production d'un médicament destiné à être utilisé dans le traitement d'un trouble du SNC.

14. Utilisation d'un composé suivant l'une quelconque des revendications 1 à 6, dans la production d'un médicament destiné à être utilisé dans le traitement de la dépression ou de l'anxiété.
